# EUROPÄISCHE PATENTSCHRIFT

(11) **EP 1 028 106 B1**
(45) Veröffentlichungstag und Bekanntmachung des Hinweises auf die Patenterteilung: **12.11.2003**
(21) Anmeldenummer: 00102786.1
(22) Anmeldetag: 10.02.2000
(51) Int. Cl.: C07C 277/02, C07C 279/14

(54) **Verfahren zur Herstellung von Guanidinderivaten**
Process for the preparation of guanidine derivatives
Procédé pour la préparation de dérivés de guanidine

(30) Priorität: 11.02.1999 DE 19905711
(43) Veröffentlichungstag der Anmeldung: 16.08.2000
(73) Patentinhaber: BASF AKTIENGESELLSCHAFT, 67056 Ludwigshafen (DE)
(72) Erfinder: Kessel, Knut, 68161 Mannheim (DE); Kluge, Michael, 67071 Lugwigshafen (DE); Bogenstätter, Thomas, 67098 Bad Dürkheim (DE); Scherr, Günter, 67065 Lugwigshafen (DE)
(74) Vertreter: Kinzebach, Werner, Dr.

(56) Entgegenhaltungen:
- EP-A- 0 754 679

## Beschreibung

Die vorliegende Erfindung betrifft ein Verfahren zur Herstellung von Guanidinderivaten der Formel I, 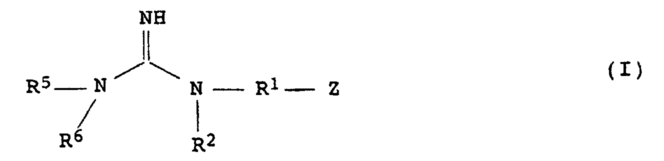 worin
- R¹: für C₁-C₈-Alkylen oder einen zweiwertigen cycloaliphatischen Rest mit 5 bis 10 Kohlenstoffatomen,
- R²: für H oder C₁-C₈-Alkyl steht, oder
- R¹ und R²: gemeinsam mit dem N-Atom, an das sie gebunden sind, für einen 5- oder 6-gliedrigen mit Z substituierten Ring stehen,
- Z: für COOR³, SO₂OR³ oder PO(OR³)(OR⁴),
- R³: jeweils unabhängig für H, ein Äquivalent eines (Erd-)Alkalimetalls,
- R⁴: für H, ein Äquivalent eines (Erd-)Alkalimetalls oder C₁-C₆-Alkyl steht, und
- R⁵ und R⁶: unabhängig für H oder C₁-C₈-Alkyl stehen,
durch Umsetzung eines Cyanamidderivats der Formel II, 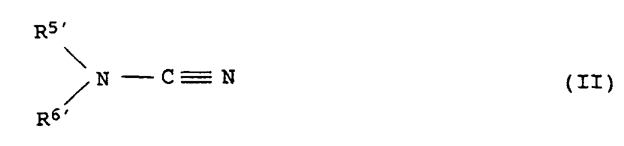 worin
- R^{5'} und R^{6'}: unabhängig für H, das Äquivalent eines (Erd-) Alkalimetalls oder für C₁-C₈-Alkyl stehen,
mit einem Amin der Formel III, 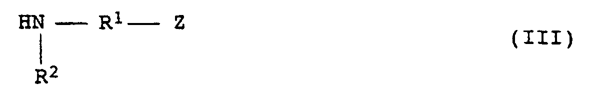 worin R¹, R² und Z die angegebene Bedeutung besitzen.

Die Erfindung betrifft insbesondere ein Verfahren zur Herstellung von Kreatin aus Cyanamid oder seinen Salzen und Sarkosinat.

Guanidinverbindungen der Formel I sind in der Natur weit verbreitet. Wichtige Vertreter dieser Substanzklasse sind beispielsweise Verbindungen wie Arginin und Kreatin. Kreatin kommt im Muskelgewebe der Wirbeltiere insbesondere als Kreatinphosphat vor und spielt eine wichtige Rolle als Energieträger der Zelle. Kreatin findet zunehmend Verwendung als Nahrungsergänzungsmittel zur Stärkung der körperlichen Leistungsfähigkeit. Außerdem findet Kreatin Verwendung zur Behandlung von Muskelfunktionsstörungen, die durch erhöhte Kreatinausscheidung im Urin gekennzeichnet sind.

In der EP-A-0754679 ist ein Verfahren zur Herstellung von Kreatin durch Umsetzung von Cyanamid mit Natrium- oder Kaliumsarkosinat beschrieben. Die Umsetzung wird absatzweise durchgeführt und es wird angegeben, dass diese in der Regel nach 2-5 h beendet ist. Die diskontinuierliche Verfahrensführung ist nachteilig, weil das absatzweise in Form einer wässrigen Suspension anfallende Reaktionsprodukt entweder diskontinuierlich nach Anfall aufgearbeitet oder in gerührten Pufferbehältern zwischengelagert werden muß. Die diskontinuierliche Synthese ist außerdem personalintensiv und fehleranfällig. Zudem tritt eine unerwünschte Minderung der Produktausbeute dadurch auf, dass die kreatingesättigten Waschwasser nicht in den Prozess zurückgeführt werden.

Der vorliegenden Erfindung liegt daher die Aufgabe zugrunde, das eingangs genannte Verfahren so weiterzubilden, dass auf einfache Weise kontinuierlich Guanidinderivate der Formel I in hoher Reinheit und hoher Ausbeute erhalten werden.

Erfindungsgemäß wird diese Aufgabe dadurch gelöst, dass das Cyanamidderivat der Formel II mit dem Amin der Formel III in Gegenwart von Impfkristallen des Guanidinderivats der Formel I kontinuierlich vereinigt werden. "Kontinuierliches Vereinigen" bedeutet, dass die Zugabe der Reaktionspartner in einer Weise erfolgt, dass das Konzentrationsverhältnis der Reaktionspartner - von der Anfahrphase gegebenenfalls abgesehen - im Reaktionsgemisch über die Dauer der Umsetzung im Wesentlichen zeitlich konstant ist. Dies ist insbesondere dann nicht der Fall, wenn ein Reaktionspartner vorgelegt wird und der andere Reaktionspartner über einen längeren Zeitraum zudosiert wird.

Das erfindungsgemäße Verfahren bedient sich des Einsatzes eines Cyanamidderivats der Formel II. Diese sind - wie im Falle des Cyanamids und seiner Salze - kommerziell erhältlich oder ihre Synthese ist dem Fachmann wohlbekannt.

R⁵ und R⁶ in der Formel I stehen unabhängig für H oder verzweigtes oder unverzweigtes C₁-C₈-Alkyl, insbesondere Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 2,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, wobei C₁-C₄-Alkyl, wie Methyl, Ethyl, Propyl, iso-Propyl, Butyl, bevorzugt ist. Vorzugsweise ist R⁵ = R⁶ = H.

R^{5'} und R^{6'} in der Formel II haben die für R⁵ bzw. R⁶ angegebene Bedeutung und können zusätzlich für das Äquivalent eines (Erd-)Alkalimetalls stehen. Besonders bevorzugt sind R^{5'} = R^{6'} = H oder R^{5'} = H und R^{6'} = Alkalimetall oder R^{5'} und R^{6'} stehen gemeinsam für ein Erdalkalimetall, insbesondere Calcium. Cyanamid ist als Cyanamidderivat der Formel II am stärksten bevorzugt.

Als weiterer Ausgangsstoff kommt ein Amin der Formel III zum Einsatz. R¹ steht für einen verzweigten oder unverzweigten C₁-C₈-Alkylenrest, vorzugsweise Methylen, Ethylen, n-Propylen, 1-Methylethylen, n-Butylen, 1-Methylpropylen, 2-Methylpropylen, 1,1-Dimethylethylen, n-Pentylen, 1-Methylbutylen, 2-Methylbutylen, 3-Methylbutylen, 2,2-Dimethylpropylen, 1-Ethylpropylen, n-Hexylen, 1,1-Dimethylpropylen, 2,2-Dimethylpropylen, 1-Methylpentylen, 2-Methylpentylen, 3-Methylpentylen, 4-Methylpentylen, 1,1-Dimethylbutylen, 1,2-Dimethylbutylen, 1,3-Dimethylbutylen, 2,2-Dimethylbutylen, 2,3-Dimethylbutylen, 3,3-Dimethylbutylen, 1-Ethylbutylen, 2-Ethylbutylen, 1,1,2-Trimethylpropylen, 1,2,2-Trimethylpropylen, 1-Ethyl-1-methylpropylen, 1-Ethyl-2-methylpropylen, n-Heptylen, n-Octylen oder einen zweiwertigen cycloaliphatischen Rest mit 5 bis 10 Kohlenstoffatomen, wie 1,2-, 1,3-, 1,4-Cyclohexylen, 1,2- oder 1,3-Cyclopentylen oder Reste folgender Struktur: 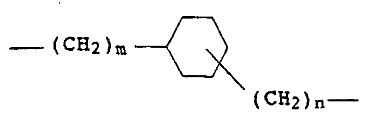 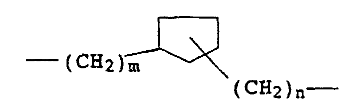 worin n und m die Werte 0, 1 oder 2 einnehmen können, mit der Maßgabe, dass n und/oder m von 0 verschieden ist. In den cycloaliphatischen Resten kann sowohl cis- als auch trans-Konfiguration vorliegen. Bevorzugt sind C₁-C₆-Alkylen, wie Methylen und Ethylen.

Der Rest R¹ kann mit einem oder mehreren, z.B. 1 bis 3, Substituenten, ausgewählt unter einer gegebenenfalls geschützten Amino-, Hydroxy- oder Cyanogruppe, substituiert sein.

R² steht für H oder C₁-C₈-Alkyl, vorzugsweise für H, Methyl oder Ethyl.

Z steht für COOR³, SO₂OR³, oder PO(OR³)(OR⁴), worin R³ jeweils unabhängig für H, ein Alkalimetall, wie Natrium oder Kalium, oder ein Äquivalent eines Erdalkalimetalls, wie Calcium, steht, und R⁴ für H, ein Alkalimetall, ein Äquivalent eines Erdalkalimetalls oder C₁-C₆-Alkyl steht.

Bei dem Amin der Formel III handelt es sich vorzugsweise um eine Aminocarbonsäure, Aminosulfonsäure oder Aminophosphonsäure oder ein Salz davon. Besonders bevorzugt sind Sarkosin, insbesondere in Form von Natrium- oder Kaliumsarkosinat, sowie Glycin, Taurin oder 4-Aminomethylcyclohexancarbonsäure.

Erfindungsgemäß werden das Cyanamid der Formel II und das Amin der Formel III in Gegenwart von Impfkristallen des Guanidinderivats der Formel I kontinuierlich miteinander in Kontakt gebracht. So kann z.B. ein Strom des Cyanamidderivats, vorzugsweise in Form einer wässrigen Lösung, mit den Impfkristallen vermischt werden und die erhaltene Suspension mit dem Amin der Formel III, vorzugsweise in Form einer wässrigen Lösung, umgesetzt werden. Andererseits kann das Amin der Formel III mit den Impfkristallen vermischt werden und anschließend mit dem Cyanamidderivat gemischt werden. In einer weiteren Alternative können die Impfkristalle in Form einer Suspension vorgehalten werden, und das Cyanamidderivat der Formel II und das Amin der Formel III werden mit den vorgehaltenen Impfkristallen vereinigt.

Das Cyanamidderivat der Formel II und das Amin der Formel III werden vorzugsweise in einem molaren Verhältnis von 2:1 bis 1:2, insbesondere von 0,8:1 bis 1,1:1 eingesetzt.

Die erfindungsgemäße Umsetzung findet in der Regel in einem wässrigen Medium, wie z.B. Wasser selbst oder einem Gemisch von Wasser mit wassermischbaren organischen Lösungsmitteln, wie Alkoholen, z.B. Methanol oder Ethanol; Aceton oder THF, statt. Das Cyanamidderivat der Formel II und das Amin der Formel III können in Form von Lösungen in Wasser oder den genannten wassermischbaren Lösungsmitteln oder Gemischen davon eingesetzt werden.

Die erfindungsgemäße Umsetzung findet vorzugsweise bei einem pH-Wert von 8 bis 12, insbesondere 9 bis 10 statt. Zur Einhaltung eines pH-Wertes im angegebenen Bereich ist es in der Regel erforderlich, in das Reaktionsmedium eine Säure oder Base einzuführen, was von der Art des eingesetzten Cyanamidderivats der Formel II und des Amins der Formel III abhängt.

Geeignete Säuren sind z.B. organische Säuren wie Ameisensäure und Essigsäure, anorganische Säuren wie Salzsäure, Schwefelsäure und Phosphorsäure, sowie insbesondere Kohlendioxid in gasförmiger oder fester Form.

Zur pH-Werteinstellung geeignete Basen sind insbesondere NaOH, KOH, LiOH oder Ca(OH)₂.

Die Einführung der Säure oder Base kann bedarfsgesteuert geschehen. Hierzu wird mittels einer kontinuierlich arbeitenden pH-Wert-Messung der aktuelle pH-Wert des Reaktionsmediums gemessen, mit einem pH-Vorgabewert verglichen und die zur pH-Wert-Korrektur notwendige Säure- oder Basenmenge dazugegeben. Der gemessene pH-Wert kann z.B. in Form eines elektronischen Signals bereitgestellt werden, der von einem elektronischen Datenprozessor mit einem Vorgabewert verglichen wird, der automatisch die erforderliche Säure- oder Basenmenge berechnet und eine Dosierungsanlage steuert.

Bei einer besonders bevorzugten Ausführungsform der Säuredosierung erfolgt die pH-Werteinstellung, indem eine wässrige Lösung des Amins der Formel III mit Kohlendioxid versetzt wird und diese Lösung zur Umsetzung mit dem Cyanamidderivat verwendet wird. Das Versetzen mit Kohlendioxid erfolgt z.B. durch Einleiten von CO₂-Gas oder Zugabe von Trockeneis. Diese Variante ist besonders bevorzugt in Fällen, in denen die eingesetzte Lösung des Amins der Formel III einen pH-Wert aufweist, der größer ist, als der angestrebte pH-Wert im Reaktionsmedium. Ein Beispiel hierfür ist eine technische Lösung von Sarkosin/Sarkosinat, die durch basische Verseifung von Sarkosinnitril hergestellt ist und in der Regel einen mehr oder weniger großen Basenüberschuß aufweist. In der mit Kohlendioxid versetzten Aminlösung stellt sich ein Gleichgewicht mit dem entsprechenden Carbamat ein. Bei der Umsetzung mit dem Cyanamidderivat der Formel II werden die freigesetzten Hydroxidionen weitestgehend durch Rückspaltung des Carbamats verbraucht, so dass sich das System selbst puffert und keine externe pH-Regelung erforderlich ist. Für den Fall, daß das Amin der Formel III Natrium- oder Kaliumsarkosinat in Form einer wässrigen Lösung ist, kann man diese mit Kohlendioxid beispielsweise auf einen pH-Wert von 8 bis 12, insbesondere von 9 bis 10, einstellen und kontinuierlich mit einem Cyanamidderivat der Formel II, z.B. Cyanamid, vereinigen.

Das erfindungsgemäße Verfahren erfolgt vorzugsweise bei. einer Temperatur von +20 bis 150°C, vorzugsweise 40 bis 100°C, insbesondere 60 bis 80°C. Die Umsetzung zwischen dem Cyanamidderivat der Formel II und dem Amin der Formel III verläuft schwach exotherm. Zur Einhaltung der angegebenen Temperatur ist es daher in der Regel erforderlich, das Reaktionsmedium zu heizen. Dies erfolgt zweckmäßigerweise über geeignete Wärmetauschvorrichtungen. Alternativ kann kontinuierlich eine Teilmenge des Reaktionsmediums über eine Temperierstrecke umgepumpt werden. Die Reaktorverweilzeiten beim erfindungsgemäßen Verfahren betragen im Allgemeinen 0,5 bis 48, bevorzugt 2 bis 36, ganz besonders bevorzugt 4 bis 24 Stunden.

Das erfindungsgemäße Verfahren kann bei Überdruck, Normaldruck oder unter Vakuum, z.B. bei einem absoluten Druck bis hinunter zu 10 mbar, durchgeführt werden. Im Allgemeinen ist das Arbeiten unter Normaldruck bevorzugt. Das Arbeiten unter Vakuum kann vorteilhaft sein, um den als Reaktionsnebenprodukt gebildeten Ammoniak aus dem Reaktionsgemisch zu entfernen.

Beim erfindungsgemäßen Verfahren werden Cyanamid der Formel II und Amin der Formel III in Gegenwart von Impfkristallen des Guanidinderivats der Formel I kontinuierlich vereinigt. Die Entnahme des gebildeten Kristallzuwachses an Guanidinderivat kann kontinuierlich oder absatzweise erfolgen.

Für die Durchführung des erfindungsgemäßen Verfahrens sind gängige Reaktoren geeignet, wie insbesondere ein kontinuierlicher Rührkesselreaktor oder eine Rührkesselkaskade. Zweckmäßigerweise wird für eine gute Durchmischung der Reaktanden im Reaktionsraum gesorgt.

Die kontinuierliche Verfahrensführung weist den Vorteil auf, dass im Reaktionsmedium im Wesentlichen konstante Konzentrationen der Reaktanden und eine im Wesentlichen konstante Menge an im Reaktionsmedium gelöstem Guanidinderivat der Formel I vorliegt. Auf diese Weise kann ein im Wesentlichen konstanter, steuerbarer Grad an Übersättigung und damit eine kontrollierte Geschwindigkeit der Kristallisation des Guanidinderivats der Formel I eingehalten werden. Durch die Steuerung der Kristallisationsgeschwindigkeit des Guanidinderivats der Formel I können Produkte hoher Reinheit mit weitgehend einheitlicher Teilchengrößenverteilung sowie mittlerer Partikelgrößen von z.B. über 150 µm erhalten werden. Hierdurch kann der Filterwiderstand der Suspension auf Werte von z.B. unter 10x10¹² mPas·m⁻² abgesenkt und praktikable Filtrationszeiten erreicht werden. In der Regel weisen die erhaltenen Kristalle eine hinreichend hohe Reinheit auf, so dass sie, gegebenenfalls nach Waschen, ohne weitere Aufreinigung z.B. als Nahrungsergänzungsmittel verwendet werden können.

In einer besonders bevorzugten Ausführungsform erfolgt die Umsetzung in einem Reaktionsraum, in den kontinuierlich Cyanamidderivat der Formel II und Amin der Formel III eingeführt werden und aus dem kontinuierlich Kristalle des Guanidinderivats der Formel I, vorzugsweise in Form einer Kristallsuspension, entnommen werden. Zweckmäßigerweise werden das Cyanamidderivat der Formel II und/oder das Amin der Formel III in Form wässriger Lösungen eingeführt. In dem Reaktionsraum müssen stets Kristalle des Guanidinderivats der Formel I vorliegen, die als Impfkristalle dienen. Dies kann z.B. dadurch erreicht werden, dass die Entnahme von Kristallen so gesteuert wird, dass ein Teil davon als Impfkristalle im Reaktionsraum zurückbleibt.

Für den Erhalt eines Produktes mit vorherbestimmbarer und einheitlicher Größenverteilung ist es allerdings bevorzugt, in den Reaktionsraum kontinuierlich Impfkristalle des Guanidinderivats der Formel I, vorzugsweise in Form einer Suspension, einzuführen. Die eingeführten Impfkristalle können eine von den entnommenen Kristallen verschiedene mittlere Kristallgröße und/oder Kristallgrößenverteilung aufweisen.

Die als Produkt entnommenen Kristalle des Guanidinderivats der Formel I können einer Größenklassierung unterworfen werden, wobei ein Feingutanteil und ein Grobgutanteil gewonnen wird und der Feingutanteil als Impfkristalle in den Reaktionsraum zurückgeführt und der Grobgutanteil als Produkt aus dem Verfahren ausgeleitet wird. Der Feingutanteil, der als Impfkristalle geeignet ist, weist z.B. eine Teilchengrößenverteilung von 1 bis <100 µm auf. Der Grobgutanteil weist z.B. eine Teilchengrößenverteilung von 100 µm bis 500 µm auf. Die Größenklassierung der Kristalle kann sowohl in suspendiertem als auch in trockenem Zustand erfolgen. Zur Klassierung im trockenen Zustand werden die Kristalle vorher von der Mutterlauge getrennt und getrocknet. Der z.B. durch Siebung gewonnene Feingutanteil kann zur Rückführung in den Reaktionsraum in einem wässrigen Medium suspendiert werden. Geeignete Vorrichtungen zur Größenklassierung sind z.B. Hydrocyclone und Naßsiebe für die Klassierung suspendierter Kristalle. Zur Klassierung getrockneter Kristalle sind z.B. Cyclone, Siebe oder Sichter geeignet.

Das Amin der Formel III kann mit Vorteil in Form einer wäßrigen Lösung, z.B. mit einer Konzentration von 85 bis 15, insbesondere 70 bis 30 Gew.-%, eingesetzt werden. Eine geeignete Lösung ist z.B. eine handelsübliche wässrige Lösung von Natriumsarkosinat mit einem Gehalt von 40 Gew.-% und einer Reinheit von 85-90 Gew.-%.

Das Einbringen des Cyanamidderivats der Formel II und/oder des Amins der Formel III oder der wässrigen Lösungen davon sowie gegebenenfalls erforderlicher Säure oder Base in das Reaktionsmedium kann zweckmäßigerweise so erfolgen, dass aus dem Reaktionsraum eine Teilmenge des Reaktionsmediums entnommen wird, mit dem Cyanamidderivat und/oder dem Amin und/oder der Säure oder Base vermischt wird und in den Reaktionsraum zurückgeführt wird. Die Entnahme, das Vermischen und Zurückführen erfolgen vorzugsweise kontinuierlich, z.B. durch Umpumpen von Reaktionsmedium über eine Dosierungs- und Mischstrecke, in der Cyanamidderivat und/oder Amin oder wässrige Lösungen davon und/oder Säure eingespeist werden. Auf diese Weise lassen sich die gewünschten Konzentrationen zweckmäßig einstellen. Lokale Konzentrationsspitzen und pH-Schwankungen können so vermieden werden.

Aus der als Produktstrom entnommenen Suspension von Kristallen des Guanidinderivats der Formel I wird das Produkt nach üblichen Verfahren, z.B. durch Filtration oder Zentrifugation, von der Mutterlauge abgetrennt. Geeignete Vorrichtungen, wie Drucknutschen, Vakuumbandfilter, Drehtrommelfilter und Schälzentrifugen sind dem Fachmann bekannt. Die abgetrennte Mutterlauge wird aus dem Verfahren ausgeleitet und z.B. verworfen. Durch Ausleiten der zurückbleibenden Mutterlauge wird eine Anhäufung von gebildeten Nebenprodukten und Verunreinigungen, die in dem eingesetzten Cyanamidderivat der Formel II und/oder dem Amin der Formel III enthalten sind, im System verhindert.

Die von der Mutterlauge abgetrennten Kristalle können, z.B. mit kaltem oder warmem Wasser, gewaschen werden. Hierzu können die Kristalle mit dem Waschmedium aufgeschlämmt und anschließend abgetrennt werden. Gemäß einem bevorzugten Aspekt der Erfindung wird das Waschmedium, welches bei der Wäsche der erhaltenen Kristalle des Guanidinderivats der Formel I anfällt, recyclisiert. Das Waschmedium kann beispielsweise zur Vorverdünnung bzw. zum Lösen des Cyanamidderivats oder des Amins verwendet werden. Das Waschmedium kann weiterhin zum Verdünnen des Reaktionsgemisches verwendet werden. Auf diese Weise wird zum einen der Wasserverbrauch minimiert, zum anderen wird der Anteil an Guanidinderivat der Formel I, der sich beim Waschen des erhaltenen Produkts im Waschmedium löst, in den Reaktionsraum zurückgeführt und bleibt im Gesamtsystem erhalten.

Die von der Mutterlauge abgetrennten und gegebenenfalls gewaschenen Kristalle des Guanidinderivats der Formel I können dann nach üblichen Verfahren getrocknet werden. Hierzu sind z.B. Stromtrockner oder Wirbelbetten geeignet. Zweckmäßigerweise werden die Kristalle auf einen Gehalt an nicht als Kristallwasser gebundener Feuchtigkeit von 5 bis 0 Gew.-%, vorzugsweise 2,5 bis 0 Gew.-%, getrocknet. Im Einzelfall kann es vorteilhaft sein, den nach der Abtrennung von der Mutterlauge erhaltenen feuchten Kristallkuchen mit bereits getrocknetem Material zu vermischen, um ein Verbacken beim Trocknen zu verhindern. Geeignete Maßnahmen zur Trockengutrückführung sind dem Fachmann bekannt.

Je nach Temperatur und Umgebungsfeuchtigkeit ist entweder die wasserfreie Form oder ein Hydrat die thermodynamisch stabilste Form des Guanidinderivats der Formel I; gegebenenfalls werden zusätzliche Hydrate mit abweichenden Zusammensetzungen als metastabile Strukturen erhalten. Im Falle des Kreatins ist das Monohydrat die übliche Handelsform; bei der Trocknung muß gegebenenfalls nach dem Fachmann bekannten Verfahren dafür gesorgt werden, dass keine unerwünschte Übertrocknung eintritt.

Die Schritte Trennung von der Mutterlauge, Waschen und Trocknen erfolgen mit Vorteil kontinuierlich in hierzu geeigneten Vorrichtungen.

Die Erfindung wird durch das folgende Beispiel näher veranschaulicht.

Beispiel - Kontinuierliche Kreatinsynthese in Gegenwart vorgelegter Kreatinkristalle

In einem doppelwandigen Glasreaktor werden 150 ml kreatingesättigtes Waschwasser und 1 g Kreatin-Monohydrat bei 75°C vorgelegt. Nun werden kontinuierlich die folgenden Ströme (i) - (iii) synchron eindosiert:
(i) 1,5 ml/min der vereinigten Waschlaugen des Vorversuchs;
(ii) 1,833 ml/min einer wässrigen Lösung des Volumens 5500 ml, die durch Eingasen von etwa 550 g Kohlendioxidgas in 5375 ml einer 40%igen technischen Lösung von Sarkosin-Natriumsalz hergestellt wurde; hierbei wird bei 75°C pH=9 erreicht. Dies entspricht innerhalb einer mittleren Verweilzeit von 10 Stunden einer Dosierung von 4,8 mol Edukt;
(iii) 0,525 ml/min einer 15°C kalten 50%igen wässrigen Lösung von Cyanamid, was innerhalb einer mittleren Verweilzeit von 10 Stunden 4,0 mol Edukt entspricht.

Es wird energisch gerührt und die Innentemperatur des Reaktors mit einem Thermostaten bei 75°C gehalten; es stellt sich ein konstanter pH-Wert von etwa 10,0 ein. Sobald der Ansatz ein Volumen von 2300 ml erreicht hat, werden über ein Ventil jeweils etwa 100 ml der Produktsuspension abgelassen. Nach 50 Stunden wird die im Reaktor verbleibende Suspension von 2300 ml Volumen auf 35°C abgekühlt und mit einem Überdruck von 1,5 bar über eine gesinterte Metalldrahtgewebe-Verbundplatte abgedrückt; der Filterwiderstand in der Drucknutsche beträgt 2x10¹² mPasm⁻². Der Reaktor wird mit 400 ml Trinkwasser ausgespült und der Filterkuchen hiermit überschichtet; mit 0,5 bar Überdruck wird alles 10 Minuten lang trockengeblasen. Der Rückstand wird mit je 350 ml Trinkwasser zweimal aufgeschlämmt und jeweils 30 Minuten intensiv gerührt; dazwischen kann leicht bis auf 10% Restfeuchte trockengesaugt werden. Die etwa 50% Feststoff enthaltende letzte Suspension wird über Nacht mit wassergesättigter Luft bei Raumtemperatur abgesaugt, bis Kreatin in Form seines Monohydrates zurückbleibt. Es werden 450 g (3,0 mol) H₂N-C(=NH)-NMe-CH₂-COOH x H₂O erhalten, was einer Ausbeute von 62,5% bezüglich Sarkosin-Natrium und 75% bezüglich Cyanamid entspricht. Der per HPLC und Titration feststellbare Gehalt an Wertprodukt ist größer als 99,5% und die Sulfatasche liegt unter 0,1%; Verbackungen des Produktes treten während des Trocknens nicht auf.

## Patentansprüche

1. Verfahren zur Herstellung von Guanidinderivaten der Formel I, 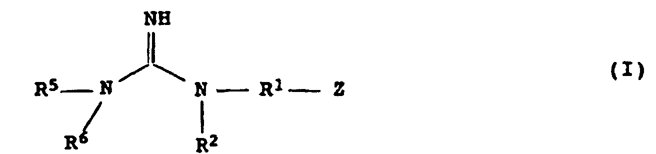 worin
R¹ für C₁-C₈-Alkylen oder einen zweiwertigen cycloaliphatischen Rest mit 5 bis 10 Kohlenstoffatomen, der mit 1 bis 3 unter einer gegebenenfalls geschützten Amino-, einer Hydroxy- oder Cyanogruppe ausgewählten Substituenten substituiert sein kann,
R² für H oder C₁-C₈-Alkyl steht, oder
R¹ und R² gemeinsam mit dem N-Atom, an das sie gebunden sind, für einen 5- oder 6-gliedrigen mit Z substituierten Ring stehen,
Z für COOR³, SO₂OR³ oder PO(OR³)(OR⁴),
R³ jeweils unabhängig für H, ein Äquivalent eines (Erd-) Alkalimetalls,
R⁴ für H, ein Äquivalent eines (Erd-) Alkalimetalls oder C₁-C₆-Alkyl steht, und
R⁵ und R⁶ unabhängig für H oder C₁-C₈-Alkyl stehen,
durch Umsetzung eines Cyanamidderivats der Formel II, 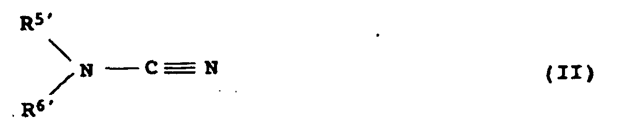 wobei
R^{5'} und R^{6'} unabhängig für H, das Äquivalent eines (Erd-)Alkalimetalls oder für C₁-C₈-Alkyl stehen,
mit einem Amin der Formel III, 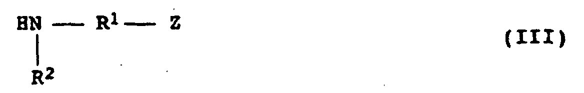 worin R¹, R² und Z die angegebene Bedeutung besitzen,
**dadurch gekennzeichnet, dass** das Cyanamidderivat der Formel II und das Amin der Formel III in Gegenwart von Impfkristallen des Guanidinderivats der Formel I kontinuierlich vereinigt werden.

2. Verfahren nach Anspruch 1, **dadurch gekennzeichnet, dass** die Umsetzung in einem Reaktionsraum erfolgt, in den kontinuierlich Cyanamidderivat der Formel II und Amin der Formel III eingeführt werden und aus dem kontinuierlich Kristalle des Guanidinderivats der Formel I entnommen werden.

3. Verfahren nach Anspruch 2, **dadurch gekennzeichnet, dass** in den Reaktionsraum kontinuierlich Impfkristalle des Guanidinderivats der Formel I eingeführt werden.

4. Verfahren nach Anspruch 3, **dadurch gekennzeichnet, dass** die entnommenen Kristalle einer Größenklassierung unterworfen werden, wobei ein Feingutanteil und ein Grobgutanteil gewonnen wird und der Feingutanteil als Impfkristalle in den Reaktionsraum zurückgeführt und der Grobgutanteil als Produkt aus dem Verfahren ausgeleitet wird.

5. Verfahren nach einem der vorhergehenden Ansprüche, **dadurch gekennzeichnet, dass** die erhaltenen Kristalle des Guanidinderivats der Formel I mit einem wässrigen Medium gewaschen werden und das Waschmedium ganz oder teilweise in das Verfahren zurückgeführt wird.

6. Verfahren nach einem der vorhergehenden Ansprüche, **dadurch gekennzeichnet, dass** die Umsetzung bei einem pH-Wert von 8 bis 12 erfolgt.

7. Verfahren nach Anspruch 6, **dadurch gekennzeichnet, dass** die Einstellung des pH-Wertes mit CO₂ erfolgt.

8. Verfahren nach einem der vorhergehenden Ansprüche, **dadurch gekennzeichnet, dass** die Umsetzung bei einer Temperatur von 20 bis 150°C erfolgt.

9. Verfahren nach einem der vorhergehenden Ansprüche, **dadurch gekennzeichnet, dass** das Cyanamidderivat der Formel II und/oder das Amin der Formel III in technischer Qualität eingesetzt werden.

## Claims

1. A process for preparing guanidine derivatives of the formula I 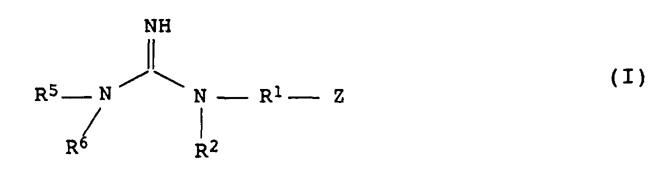 in which
R¹ is C₁-C₈-alkylene or a divalent cycloaliphatic radical having 5 to 10 carbon atoms, which may be substituted by 1 to 3 substituents selected from an optionally protected amino, hydroxyl or cyano group,
R² is H or C₁-C₈-alkyl, or
R¹ and R² represent, together with the N atom to which they are bonded, a 5- or 6-membered Z-substituted ring,
Z is COOR³, SO₂OR³ or PO(OR³)(OR⁴),
R³ is in each case independently H, one equivalent of an alkali metal or alkaline earth metal,
R⁴ is H, one equivalent of an alkali metal or alkaline earth metal or C₁-C₆-alkyl, and
R⁵ and R⁶ are independently H or C₁-C₈-alkyl,
by reacting a cyanamide derivative of the formula II 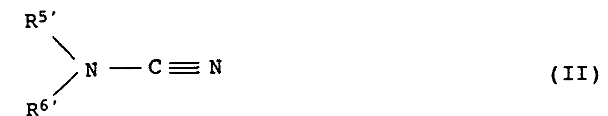 in which
R^{5'} and R^{6'} are independently H, the equivalent of an alkali metal or alkaline earth metal or C₁-C₈-alkyl,
with an amine of the formula III 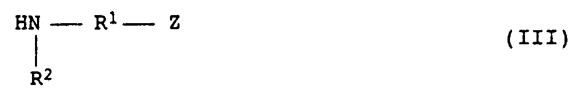 in which R¹, R² and Z have the stated meanings,
where the cyanamide derivative of the formula II and the amine of the formula III are continuously combined in the presence of seed crystals of the guanidine derivative of the formula I.

2. A process as claimed in claim 1, wherein the reaction takes place in a reaction chamber into which cyanamide derivative of the formula II and amine of the formula III are continuously introduced, and from which crystals of the guanidine derivative of the formula I are continuously removed.

3. A process as claimed in claim 2, wherein seed crystals of the guanidine derivative of the formula I are continuously introduced into the reaction chamber.

4. A process as claimed in claim 3, wherein the removed crystals are subjected to a size classification to obtain a fine material fraction and a coarse material fraction, with the fine material fraction being returned as seed crystals to the reaction chamber, and the coarse material fraction being discharged from the process as product.

5. A process as claimed in any of the preceding claims, wherein the resulting crystals of the guanidine derivative of the formula I are washed with an aqueous medium, and the washing medium is wholly or partly returned to the process.

6. A process as claimed in any of the preceding claims, wherein the reaction takes place at a pH of from 8 to 12.

7. A process as claimed in claim 6, wherein the pH is adjusted with CO₂.

8. A process as claimed in any of the preceding claims, wherein the reaction takes place at a temperature of from 20 to 150°C.

9. A process as claimed in any of the preceding claims, wherein the cyanamide derivative of the formula II and/or the amine of the formula III are employed in technical quality.

## Revendications

1. Procédé pour la préparation de dérivés de guanidine de formule I, 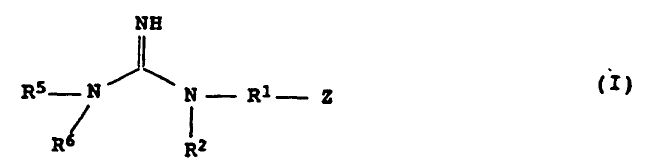 où
R¹ désigne un groupe alkylène en C₁-C₈ ou un reste cycloaliphatique bivalent ayant 5 à 10 atomes de carbone, qui peut être substitué par 1 à 3 substituants choisis parmi un groupe amino éventuellement protégé, un groupe hydroxy ou un groupe cyano,
R² représente H ou un alkyle en C₁-C₈, ou
R¹ et R² désignent, conjointement avec l'atome N auquel ils sont liés, un cycle à 5 ou 6 chaînons substitué par Z,
Z désigne COOR³, SO₂OR³ ou PO(OR³)(OR⁴),
R³ à chaque fois indépendamment, représente H ou un équivalent d'un métal alcalin ou alcalino-terreux,
R⁴ désigne H, un équivalent d'un métal alcalin ou alcalino-terreux, ou un alkyle en C₁-C₆, et
R⁵ et R⁶ représentent, indépendamment, H ou un alkyle en C₁-C₈,
par réaction d'un dérivé de cyanamide de formule II, 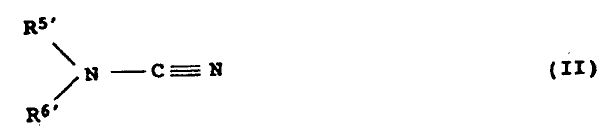 tandis que R^{5'} et R^{6'} désignent, indépendamment, H, l'équivalent d'un métal alcalin ou alcalino-terreux ou un alkyle en C₁-C₈, avec une amine de formule III, 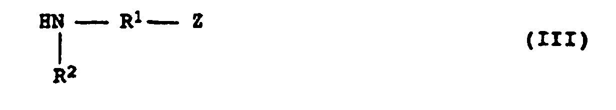 où R¹, R² et Z possèdent la signification indiquée,
**caractérisé par le fait qu'**on combine le dérivé de cyanamide de formule II et l'amine de formule III en présence de germes du dérivé de guanidine de formule I, en continu.

2. Procédé selon la revendication 1, **caractérisé par le fait qu'**on effectue la réaction dans un espace de réaction dans lequel on introduit en continu le dérivé de cyanamide de formule II et l'amine de formule III et d'où on soutire en continu des cristaux du dérivé de guanidine de formule I.

3. Procédé selon la revendication 2, **caractérisé par le fait qu'**on introduit en continu dans l'espace de réaction des germes du dérivé de guanidine de formule I.

4. Procédé selon la revendication 3, **caractérisé par le fait qu'**on soumet les cristaux prélevés à un tri granulométrique, tandis qu'on recueille une partie de matières fines et une partie de matières grossières et on recycle la partie de matières fines sous forme de germes dans l'espace de réaction et la partie de matières grossières est extraite du procédé en tant que produit.

5. Procédé selon l'une des revendications précédentes, **caractérisé par le fait que** les cristaux de dérivé de guanidine de formule I obtenus sont lavés avec un milieu aqueux et le milieu de lavage est recyclé partiellement ou en totalité dans le procédé.

6. Procédé selon l'une des revendications précédentes, **caractérisé par le fait que** la réaction s'effectue à un pH de 8 à 12.

7. Procédé selon la revendication 6, **caractérisé par le fait que** l'ajustement du pH s'effectue avec du CO₂.

8. Procédé selon l'une des revendications précédentes, **caractérisé par le fait que** la réaction s'effectue à une température de 20 à 150°C.

9. Procédé selon l'une des revendications précédentes, **caractérisé par le fait que** le dérivé de cyanamide de formule II et/ou l'amine de formule III sont utilisés dans une qualité technique.
